(19) Europäisches Patentamt
European Patent Office
Office européen des brevets

(11) Numéro de publication : **0 498 705 A1**

(12) # DEMANDE DE BREVET EUROPEEN

(21) Numéro de dépôt : **92400267.8**

(51) Int. Cl.[5] : **C07C 217/34,** C12N 9/18

(22) Date de dépôt : **03.02.92**

(30) Priorité : **08.02.91 FR 9101426**

(43) Date de publication de la demande :
**12.08.92 Bulletin 92/33**

(84) Etats contractants désignés :
**AT BE CH DE DK ES FR GB GR IT LI LU MC NL PT SE**

(71) Demandeur : **SYNTHELABO**
**22, Avenue Galilée**
**F-92350 Le Plessis Robinson (FR)**

(72) Inventeur : **Binet, Jean**
**13, rue du Faubourg Saint-Nicolas**
**F-21121 Fontaine Les Dijon (FR)**
Inventeur : **Manoury, Philippe**
**L'Orée de Verrières, 38 Avenue des Vaupépins**
**F-91370 Verrières-le-Buisson (FR)**
Inventeur : **Zard, Lydia**
**6, Impasse des Quatre Vents La Croix Audierne**
**F-91190 Gif/Yvette (FR)**
Inventeur : **Perard, Serge**
**80, rue de Plaisance**
**F-94130 Nogent-sur-Marne (FR)**
Inventeur : **Rossey, Guy**
**10, rue Paul Verlaine**
**F-78960 Voisins-le-Bretonneux (FR)**

(74) Mandataire : **Thouret-Lemaitre, Elisabeth et al**
**SYNTHELABO, 22, avenue Galilée, B.P. 72**
**F-92350 Le Plessis-Robinson Cédex (FR)**

(54) **Dérivés d'éthers de phénols, leur préparation et leur application en thérapeutique.**

(57) Isomères (*R*)(+) et (*S*)(-) du 1-[4-[2-(cyclopropylméthoxy) éthoxy]phénoxy]-3-[(1-méthyléthyl)amino]propan-2-ol et leurs sels d'addition aux acides pharmaceutiquement acceptables.
Application en thérapeutique.

EP 0 498 705 A1

La présente invention a pour objet des dérivés d'éthers de phénols, leur préparation et leur application en thérapeutique.

Dans son brevet français 75.33899 la Demanderesse a décrit des éthers de phénols substitués, utilisables en thérapeutique dans le domaine cardiovasculaire.

Parmi ces composés, le composé de formule

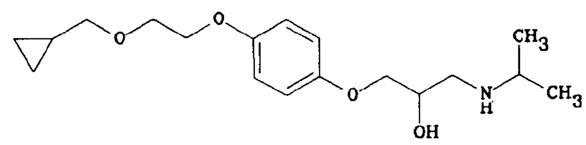

composé de l'exemple 1, et ses sels pharmaceutiquement acceptables se sont révélés intéressants.

La présente invention a pour objet les énantiomères (*R*)(+) et (*S*)(-) de ce composé, le 1-[4-[2-(cyclopropylméthoxy)éthoxy]phénoxy]-3-[(1-méthyléthyl)amino]propan-2-ol ou cicloprolol, leurs sels pharmaceutiquement acceptables, leur préparation et leur application en thérapeutique.

Les énantiomères du cicloprolol peuvent être préparés, selon l'invention, soit par synthèse chimique stéréospécifique, soit par synthèse enzymatique stéréospécifique.

<u>Synthèse chimique stéréospécifique</u>

L'énantiomère (*S*) du cicloprolol est préparé, selon l'invention, à partir du (5*S*)-3-(1-méthyléthyl)-2-phényloxazolidine-5-méthanol que l'on transforme en 4-méthylbenzènesulfonate, composé que l'on fait réagir avec le 4-[2-(cyclopropylméthoxy)éthoxy]phénol selon le schéma 1 suivant:

Schéma 1

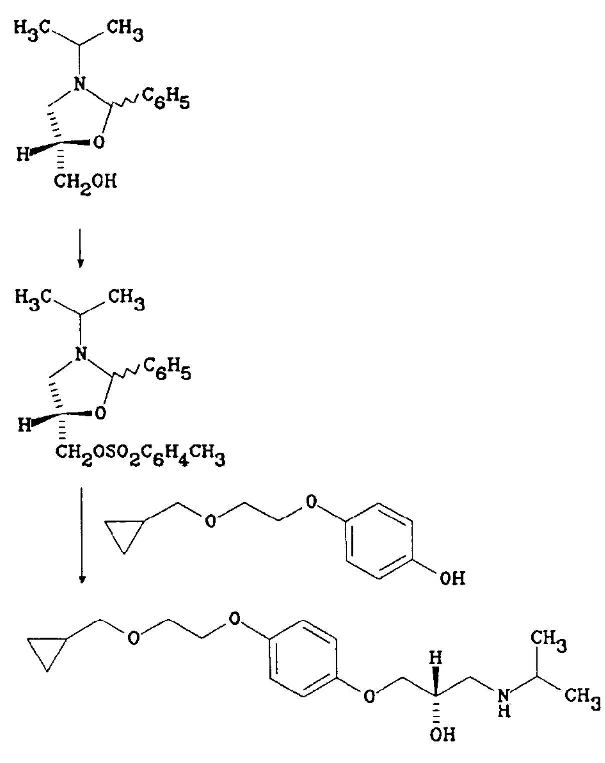

Le (5*S*)-3-(1-méthyléthyl)-2-phényloxazolidine-5-méthanol est décrit par Baldwin et coll, *J. Med. Chem.* **1979,** *22*, n° 11.

Le 4-[2-(cyclopropylméthoxy)éthoxy]phénol est décrit dans le brevet français 75.33899 de la demanderesse.

L'énantiomère (*R*) du cicloprolol est préparé, selon l'invention, à partir du 4-méthylbenzènesulfonate de [(*R*)-2,2-diméthyldioxolan-4-yl]méthyle selon le schéma 2 suivant:

Schéma 2

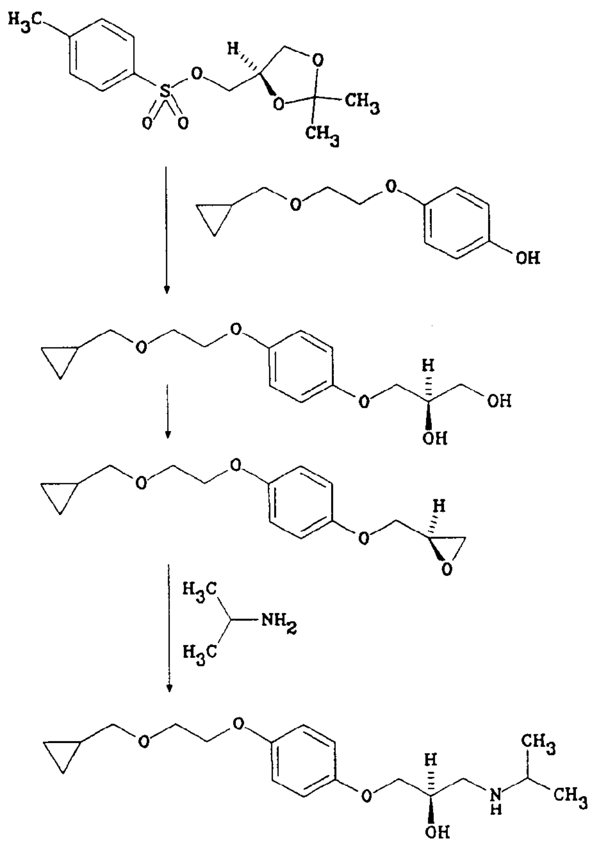

Synthèse enzymatique stéréospécifique

Le procédé de synthèse enzymatique stéréospécifique selon l'invention permet la résolution du 1-chloro-3-[4-[2-(cyclopropylméthoxy)éthoxy]phénoxy]propan-2-ol et de son ester O-acétylé. Il utilise une lipase, par exemple une lipase issue de Candida cylindricae, de Mucor javanicus, de Mucor miehei ou de Pseudomonas fluorescens. Cette dernière lipase est particulièrement utilisée, car elle permet d'obtenir des excès énantiomèriques de 96 à 99 % pour les deux énantiomères du cicloprolol. Les lipases peuvent être éventuellement immobilisées sur support.

Dans une première étape, selon le schéma suivant,

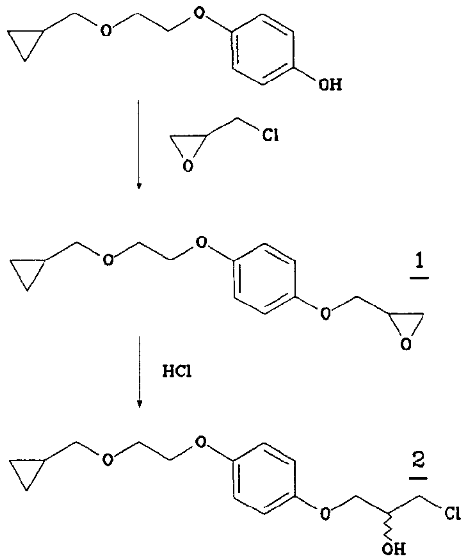


on condense le 4-[2-(cyclopropylméthoxy)éthoxy]phénol avec l'épichlorhydrine en milieu basique. On obtient un mélange de [[4-[2-(cyclopropylméthoxy)éthoxy]phénoxy]méthyl]oxirane (1) et de 1-chloro-3-[4-[2-(cyclopropylméthoxy)éthoxy]phénoxy]propan-2-ol (2). On traite le mélange par de l'acide chlorhydrique concentré pour obtenir uniquement le composé (2).

Dans une deuxième étape,

1. soit on acétyle le composé (2), dans des conditions classiques d'acétylation, selon le schéma suivant,

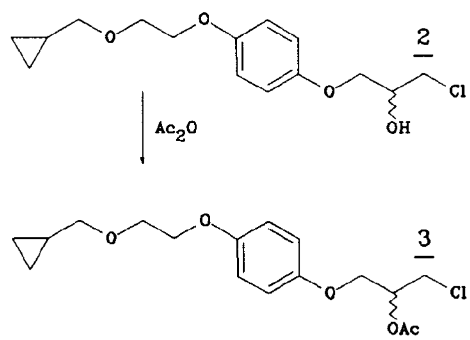


et on obtient l'acétate de 1-chloro-3-[4-[2-(cyclopropylméthoxy)éthoxy]phénoxy]prop-2-yle (3). On hydrolyse l'ester de formule (3), brut ou purifié, en présence de lipase issue de Pseudomonas fluorescens selon le schéma suivant,

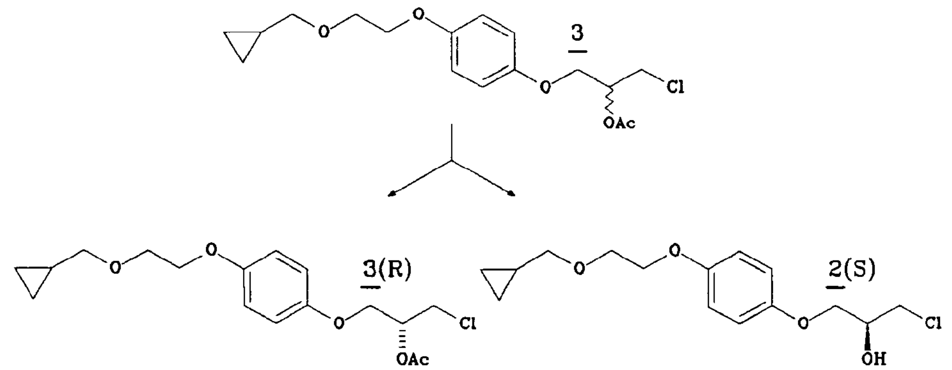
3
3(R)
2(S)

Cette hydrolyse est réalisée dans un solvant organique tel que l'éther diisopropylique, le terbutyléther, le toluène ou un solvant halogéné comme le dichlorométhane ou le chloroforme. On obtient un mélange de (*S*)-1-chloro-3-[4-[2-(cyclopropylméthoxy)éthoxy]phénoxy]propan-2-ol 2(S) et d'acétate de (*R*)-1-chloro-3-[4-[2-(cyclopropylméthoxy)éthoxy]phénoxy]prop-2-yle 3(R), que l'on peut séparer par chromatographie sur colonne de gel de silice ou par traitement par de l'anhydride succinique en présence de triéthylamine selon le schéma suivant,

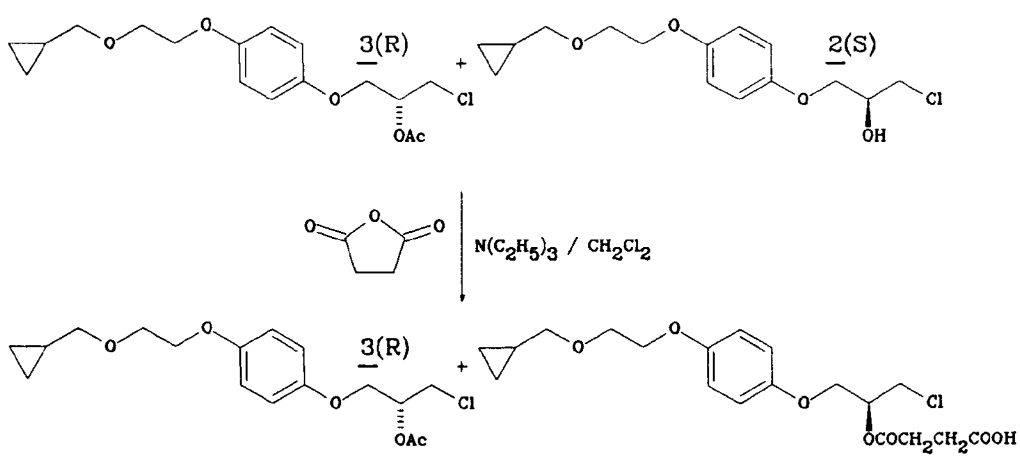

3(R)
+
2(S)
3(R)
+

2. soit on acétyle le composé (2) en présence de la lipase issue de Pseudomonas fluorescens selon le schéma suivant,

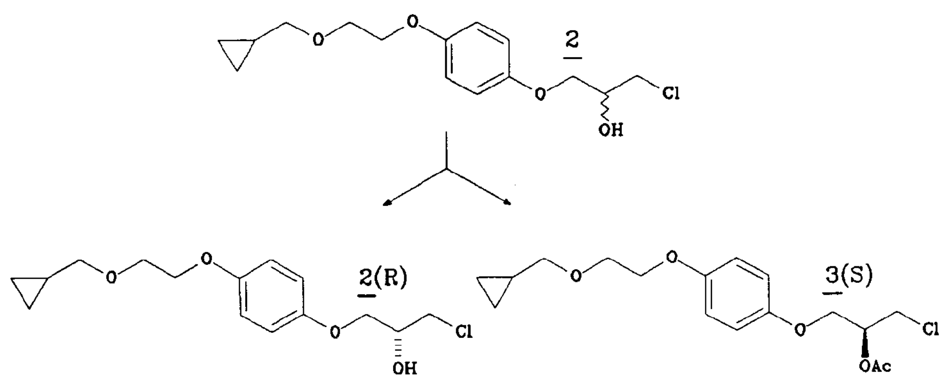
2
2(R)
3(S)

La réaction est effectuée dans un solvant organique tel que l'éther diisopropylique, le terbutyléther, le toluène ou un solvant halogéné comme le dichlorométhane ou le chloroforme, en présence d'un agent acétylant comme l'acétate de vinyle, l'acétate d'isopropényle, l'acétate d'éthyle ou l'anhydride acétique.
Le (*R*)-1-chloro-3-[4-[2-(cyclopropylméthoxy)éthoxy]phénoxy]propan-2-ol 2(*R*) et l'acétate de (*S*)-1-chloro-3-[4-[2-(cyclopropylméthoxy)éthoxy]phénoxy]prop-2-yle 3(*S*) sont séparés par chromatographie sur colonne de gel de silice.
Dans une troisième étape, on transforme le composé 2(*R*) en (*S*)-cicloprolol selon le schéma suivant,

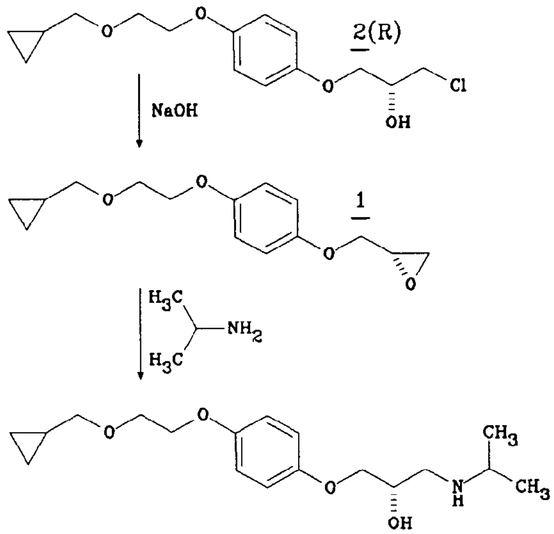

2(R)
1

On forme, à partir du composé 2(*R*), un époxyde en milieu basique que l'on fait réagir avec de l'isopropylamine. De la même manière, on obtient le (*R*)-cicloprolol à partir du composé 2(*S*).
Si l'on part des acétates 3(*R*) et 3(*S*), alors on effectue d'abord, in situ, une hydrolyse des esters avant de former les époxydes.

Les exemples suivants illustrent l'invention.
Les analyses confirment la structure des composés.

Exemple 1

(*S*)-(-)-1-[4-[2-(cyclopropylméthoxy)éthoxy]phénoxy]-3-[(1-méthyléthyl)amino]propan-2-ol et son hémifumarate.

1.1. 4-méthylbenzènesulfonate de [(5*S*)-3-(1-méthyléthyl)-2-phényloxazolidin-5-yl]méthyle.

On introduit, par portions, 7,6 g (0,04 mole) de chlorure de 4-méthylbenzènesulfonyle à une solution de 8,8 g (0,04 mole) de (5*S*)-3-(1-méthyléthyl)-2-phényloxazolidine-5-méthanol dans 15 ml de pyridine, à une température allant de 0 à 5°C. On agite le mélange réactionnel pendant 2 h en le laissant revenir à la température ambiante, puis on le refroidit par un bain de glace. On ajoute alors 5,5 g (0,04 mole) de carbonate de potassium en solution dans 25 ml d'eau. On extrait le mélange avec du dichlorométhane. On sèche la phase organique sur du sulfate de magnésium, on filtre et on évapore à sec. On recueille 15,1 g d'huile.

1.2. (*S*)-(-)-1-[4-[2-cyclopropylméthoxy)éthoxy]phénoxy]-3-[1-méthyléthyl)amino]propan-2-ol et son hémifumarate.

On agite 8,3 g (0,04 mole) de 4-(2(cyclopropylméthoxy)éthoxy]phénol avec une suspension de 2 g (0,04 mole) d'hydrure de sodium à 50 % dans 50 ml de diméthylformamide jusqu'à ce que le dégagement d'hydrogène ait cessé. On refroidit alors le mélange à l'aide d'un bain d'eau glacée puis on ajoute goutte à goutte 15,1 g de l'huile obtenue précédemment sous 1.1 en solution dans 25 ml de diméthylformamide puis on chauffe à 50-60°C pendant 6 h. On refroidit le mélange, on verse sur de l'eau et on extrait avec de l'éther. On lave la

phase organique avec de l'eau, on la sèche sur du sulfate de magnésium, on filtre et on évapore.
On reprend l'huile obtenue après évaporation par 50 ml d'eau, on ajoute 15 ml d'acide chlorhydrique concentré en refroidissant et on laisse le mélange une nuit au repos. On extrait le benzaldéhyde formé avec de l'éther, puis on alcalinise la phase aqueuse avec de la soude et on extrait avec de l'éther. On lave la phase organique avec de l'eau, on sèche, on filtre et on évapore. L'huile résiduelle est purifiée par chromatographie sur gel de silice en éluant par un mélange chloroforme/méthanol (90/10). Après évaporation, on obtient un produit huileux que l'on purifie dans de l'éther en présence de noir animal. On filtre et on évapore l'éther. On obtient 1,5 g d'huile à partir de laquelle on prépare le fumarate dans un mélange acétone/éther. On fait recristalliser ce composé dans de l'acétate d'éthyle.
Point de fusion = 89-92°C
$[\alpha]^{20}_D$ = -15,8° (c = 0,855 ; méthanol)

Exemple 2

(*R*)-(+)-1-[4-[2(cyclopropylméthoxy)éthoxy]phénoxy]-3-[(1-méthyléthyl)amino]propan-2-ol.

2.1. 4-méthylbenzènesulfonate de [(*R*)-2,2-diméthyldioxolan-4-yl]méthyle.

A 14,4 g de (*S*)-2,2-diméthyldioxolane-4-méthanol, en solution dans 60 ml de pyridine on ajoute, par portions, 20,8 g de chlorure de 4-méthylbenzène sulfonyle et on laisse le mélange réactionnel pendant 16 h à 0°C. On dilue avec de l'éther, on lave avec de l'acide chlorhydrique 1 N jusgu'à pH acide des eaux de lavage, on lave avec de l'eau puis avec une solution saturée d'hydrogénocarbonate de sodium et enfin avec de l'eau. On sèche, on filtre et on évapore.

2.2. (*R*)-(-)-3-[4-[2-(cyclopropylméthoxy)éthoxy]phénoxy]propane-1,2-diol.

On prépare une solution de 20,8 g (0,1 mole) de 4-[2-(cyclopropylméthoxy)éthoxy]phénol et de 4 g (0,1 mole) d'hydroxyde de sodium dans 100 ml d'éthanol. On ajoute alors 14,3 g (0,05 mole) du tosylate obtenu sous 2.1 et on porte le mélange réactionnel à la température de reflux pendant 24 h. On évapore à sec, on reprend avec un mélange d'eau et d'éther. On lave la phase éthérée avec de la soude N puis avec de l'eau. On sèche sur du sulfate de magnésium, on filtre et on évapore. On obtient 14,5 g de (*S*)-4-[[4-[2-(cyclopropylméthoxy)éthoxy]phénoxy]méthyl]dioxolane sous forme d'une huile qui cristallise dans le temps. On solubilise 14,5 g (0,045 mole) de l'huile obtenue dans 100 ml d'acide chlorhydrique 2,5 N et la quantité suffisante d'acétone (150 ml). On laisse le mélange réactionnel la nuit au repos. On évapore et on reprend avec de l'éther. On lave la phase éthérée avec de la soude 1 N puis avec de l'eau. On sèche, on filtre et on évapore. Le produit cristallise dans de l'éther de pétrole. On le fait recristalliser dans de l'éther diisopropylique.
Point de fusion = 66-67°C
$[\alpha]^{25}_D$ = -5,6° (c = 2,2 ; méthanol)

2.3. (*R*)-2-[[4-[2-(cyclopropylméthoxy)éthoxy]phénoxy]méthyl]oxirane.

On refroidit, à 0-5°C, 8,4 g (0,03 mole) du composé obtenu sous 2.2 dans 30 ml de pyridine et on ajoute lentement, à une température d'environ 5°C, 6,3 g (0,033 mole) de chlorure de 4-méthylbenzènesulfonyle en solution dans 100 ml de benzène. On laisse le mélange 48 h sous agitation, on verse le mélange sur 400 ml d'acide chlorhydrique 1 N et on extrait avec de l'éther. On lave la phase éthérée avec de l'eau, on sèche, on filtre et on évapore. On chromatographie l'huile résiduelle en éluant par un mélange dichlorométhane/acétone (95/5) et on recueille, après évaporation, 9 g de 4-méthylbenzènesulfonate de (*R*)-3-[4-[2-(cyclopropylméthoxy)éthoxy]phénoxy]-2-hydroxypropyle sous forme d'huile. A une solution de 9 g (0,02 mole) du composé obtenu précédemment dans 30 ml d'éther et 50 ml de méthanol, refroidie à environ 5°C, on ajoute, par portions, 0,5 g (0,0206 mole) de sodium. On laisse une nuit au repos et on ajoute de la glace. On reprend avec un peu d'eau, on extrait avec du dichlorométhane. On lave à l'eau, on sèche, on filtre et on évapore. On obtient une huile que l'on utilise à l'état brut dans la réaction suivante.

2.4. (*R*)-(+)-1-[4-[2-(cyclopropylméthoxy)éthoxy]phénoxy]-3-[(1-méthyléthyl)amino]propan-2-ol.

On agite à la température ambiante, pendant 3 jours, 5,4 g du composé obtenu sous 2.3 en solution dans 50 ml d'isopropylamile, puis on porte le mélange au reflux pendant 16 h et on laisse une nuit au repos. On évapore à sec.

On reprend par de l'éther et on extrait avec de l'eau acidulée. On alcalinise la phase aqueuse avec de la soude concentrée en refroidissant, puis on extrait 2 fois avec de l'éther. On lave la phase éthérée avec de l'eau, on sèche, on filtre et on évapore. On recueille 4,6 g d'huile.
On prépare l'hémifumarate à partir de l'huile et de 0,9 g d'acide fumarique dans 50 ml d'acétone et 28 ml d'éther. On le fait recristalliser dans 50 ml de méthyléthylcétone .
Point de fusion = 104-106°C
$[\alpha]^{25}_D$ = +15,4° (c = 1,78 ; méthanol)

Exemple 3

(*R*)-1-[4-[2-(cyclopropylméthoxy)éthoxy]phénoxy]-3-[(1-méthyléthyl)amino]propan-2-ol ou (*R*)-cicloprolol.

3.1. 1-chloro-3-[4-[2-(cyclopropylméthoxy)éthoxy]phénoxy]propan-2-ol.

A 104 g (0,5 mole) de 4-[2-(cyclopropylméthoxy)éthoxy]phénol en suspension dans 500 ml d'eau, on ajoute une solution de 21 g (0,525 mole) d'hydroxyde de sodium dans 250 ml d'eau. Après 1 heure 30 d'agitation à 25°C, on ajoute 141 ml (1,8 moles) de chlorométhyloxirane (épichlorhydrine) et on laisse sous agitation pendant une nuit. On ajoute 50 ml (0,64 mole) d'épichlorhydrine et on chauffe, à 50°C, pendant 4 heures. A la fin de la réaction, on décante et on dilue la phase inférieure avec du dichlorométhane. On la lave avec une solution d'hydroxyde de sodium à 5 % puis avec de l'eau saturée en chlorure de sodium. On la sèche sur du sulfate de magnésium et on évapore sous vide. On obtient 187 g d'huile noire, mélange des composés (1) et(2).
On le met en suspension dans 350 ml d'acide chlorhydrique à 37 %. Après 1 heure d'agitation à 25°C, on extrait le milieu réactionnel, plusieurs fois, par du dichlorométhane. On rassemble les phases organiques, on les lave à l'eau jusqu'à neutralité, on les sèche sur du sulfate de magnésium et on évapore sous vide. On obtient 194 g du composé (2) sous la forme d'une huile.
On purifie une fraction du composé (2) ainsi obtenu par chromatographie sur colonne de gel de silice, en éluant par un mélange dichlorométhane/méthanol (95/5). On obtient le composé (2) pur sous la forme d'une poudre blanche.
Point de fusion = 35°C

3.2. acétate de 1-chloro-3-[4-[2-(cyclopropylméthoxy)éthoxy]phénoxy]prop-2-yle.

On agite une solution contenant 1,5 g (5 mmoles) de composé (2), 1,087 ml d'anhydride acétique et 1,284 ml de triéthylamine dans 12 ml de dichlorométhane, pendant 20 heures, à 25°C. A la fin de la réaction, on hydrolyse à froid et on extrait le milieu réactionnel par du dichlorométhane. On récupère la phase organique et on la lave successivement par une solution d'acide chlorhydrique à 10 %, par une solution d'hydrogénocarbonate de sodium puis par une solution saturée en chlorure de sodium. On la sèche sur du sulfate de magnésium et on évapore sous vide. On obtient 1,88 g du composé (3) sous forme d'une huile incolore. Rendement quantitatif

3.3. (*S*)-1-chloro-3-[4-[2-(cyclopropylméthoxy)éthoxy]phénoxy]propan-2-ol.

On dissout 0,684 g (2 mmoles) du composé(3), dans un mélange contenant 4 ml de tampon $KH_2PO_4$ 0,01 M à pH = 7,3 et 4 ml d'éther diisopropylique. On ajoute 0,04 g de lipase de Pseudomonas fluorescens et on suit la réaction au pH-stat. On maintient le pH constant par une solution de soude 1 M. Au bout de 7 heures, après consommation d'un demi-équivalent de soude, on extrait le milieu réactionnel par de l'éther diéthylique. On recueille la phase organique et on la lave par une solution saturée en chlorure de sodium. On la sèche sur du sulfate de magnésium et on évapore sous vide. On obtient 0,6 g d'un mélange de 2(*S*) et 3(*R*).
On sépare les deux produits par chromatographie sur colonne de gel de silice en éluant par un mélange cyclohexane/acétate d'éthyle (3/1). On obtient 0,270 g du composé 2(*S*), sous forme d'une huile incolore, et 0,288 g du composé 3(*R*), également sous forme d'une huile incolore. Ces composés présentent respectivement des excès énantiomériques de 96,6 % et de 99,7 %.
2(*S*): $[\alpha]^{20}_D$ = +0,42° (c= 5 ; chloroforme)
3(*R*): $[\alpha]^{20}_D$ = -23,2° (c= 1; chloroforme)

3.4. (*R*)-1-[4-[2-(cyclopropylméthoxy)éthoxy]phénoxy]-3-[(1-méthyléthyl)amino]propan-2-ol ou (*R*)-cicloprolol.

On dissout 0,084 g du composé 2(*S*) dans 2 ml de dichlorométhane. On ajoute 2 ml d'une solution d'hydroxyde de sodium à 30 % et on agite une nuit à 20°C. On extrait le milieu réactionnel par du dichlorométhane.

thane, on sèche et on évapore sous vide. On obtient 0,076 g d'époxyde que l'on dissout dans 0,5 ml d'éthanol absolu. On ajoute une solution de 0,12 ml d'isopropylamine dans 1 ml d'éthanol absolu. On agite à 80°C pendant 80 heures et on évapore à sec. On obtient 0,08 g de (*R*)-cicloprolol base sous forme d'une huile. Son excès énantiomèrique est de 94 %.

Exemple 4

(*R*)-1-chloro-3-[4-[2-(cyclopropylméthoxy)éthoxy]phénoxy]propan-2-ol 2(*R*) et acétate de (*S*)-1-chloro-3-[4-[2-(cyclopropylméthoxy)éthoxy]phénoxy]prop-2-yle 3(*S*).

On dissout 0,6 g (2 mmoles) de composé 2 purifié dans 24 ml d'éther diisopropylique. On ajoute 0,08 g de lipase de Pseudomonas fluorescens et 0,44 ml (5 mmoles) d'acétate d'isopropényle. On agite à 20°C pendant 6 jours en suivant la formation de l'ester. On élimine l'enzyme par filtration et on évapore le solvant sous vide. On obtient 0,71 g d'un mélange de 2(*R*) et 3(*S*) que l'on sépare par chromatographie sur colonne de gel de silice.
On obtient 0,279 g du composé 2(*R*), sous forme d'une huile incolore et 0,350 g du composé 3(*S*), sous forme d'une huile incolore. Les excès énantiomériques respectifs de ces composés sont de 99,7 % et de 92,3 %.
2(*R*): $[\alpha]^{20}_D$ = -0,42° (c= 5 ; chloroforme)
3(*S*): $[\alpha]^{20}_D$ = +20,54° (c= 1,48 ; chloroforme)

Exemple 5

(*S*)-1-[4-[2-(cyclopropylméthoxy)éthoxy]phénoxy]-3-[(1-méthyléthyl)amino]propan-2-ol ou (*S*)-cicloprolol.

5.1. séparation du mélange des composés 2(*R*) et 3(*S*)

A 1 g de mélange des composés 2(*R*) et 3(*S*), en solution dans 25 ml de dichlorométhane, on ajoute 0,45 g (4,5 mmoles) d'anhydride succinique et 0,22 ml de triéthylamine. On agite la solution à 20°C pendant 96 heures en présence de tamis moléculaires de 3 Ängstroms. On évapore le solvant et on reprend le résidu dans 25 ml d'éther diéthylique et 10 ml d'eau. On ajuste le pH à 9 avec une solution d'hydroxyde de sodium 4 M, on agite et on décante. On récupère la phase organique, on la lave avec 10 ml d'eau et avec une solution aqueuse saturée en chlorure de sodium. On la sèche sur du sulfate de magnésium et on évapore sous vide. On obtient 0,413 mg du composé 3(*R*).

5.2. (*S*)-1-[4-[2-(cyclopropylméthoxy)éthoxy]phénoxy]-3-[(1-méthyléthyl)amino]propan-2-ol ou (*S*)-cicloprolol.

A 0,210 g du composé 3(*R*) en solution dans un mélange contenant 10 ml d'hexane et 5 ml d'eau, on ajoute 0,5 ml d'une solution aqueuse d'hydroxyde de sodium à 30 %. On agite une nuit à 20°C. On extrait avec de l'éther diéthylique et on récupère la phase organique. On la lave avec une solution aqueuse saturée en chlorure de sodium, on la sèche sur du sulfate de magnésium et on évapore sous vide. On obtient 0,161 g d'époxyde.
Rendement = 99 %
On dissout l'époxyde obtenu dans 1,3 ml d'éthanol absolu et on ajoute une solution de 0,285 ml d'isopropylamine dans 2,4 ml d'éthanol absolu. On agite, sous argon, pendant 8 heures, à 40°C. On évapore puis on reprend par de l'éther. On obtient le cicloprolol sous forme de chlorhydrate par extraction avec une solution d'acide chlorhydrique à 10 %. On alcalinise la phase aqueuse avec une solution d'hydroxyde de sodium à 30 % et on l'extrait par du dichlorométhane. On la sèche sur du sulfate de magnésium et on évapore. On obtient 141 mg de (*S*)-cicloprolol sous forme de base. Ce composé présente un excès énantiomérique de 95 %.
On forme le chlorhydrate correspondant en ajoutant 4,38 ml d'une solution d'acide chlorhydrique 1 N dans l'isopropanol. Après évaporation de l'isopropanol, on fait cristalliser le produit dans l'éther diéthylique. On obtient 0,127 g de produit ayant un excès énantiomèrique de 95 %.
Point de fusion = 74-75°C
$[\alpha]^{20}_D$ = -13,5°C (c = 1 ; eau)

Les énantiomères du cicloprolol ont été testés en pharmacologie où leurs propriétés dans le domaine cardiovasculaire ont pu être démontrées.

Les résultats pharmacologiques obtenus montrent que le (*S*)-cicloprolol possède des propriétés agonistes partielles au niveau des récepteurs $\beta_1$-adrénergiques.
Ce composé possède un potentiel clinique dans le traitement de l'insuffisance cardiaque. L'activité stimulante cardiaque du cicloprolol a été confirmée chez l'homme.

**1. Efficacité des isomères (*S*)(-) et (*R*)(+) du cicloprolol en tant que bloquants des récepteurs $\beta_1$ adrénergiques dans des conditions in vitro.**

Préparation des oreillettes droites.

Des cobayes mâles (race tricolore : élevage LESSIEUX) pesant 200-250 g sont sacrifiés par dislocation cervicale. Les coeurs sont rapidement retirés de la cage thoracique et placés dans une solution de Krebs dans laquelle barbote un mélange de 95 % de $O_2$ et 5 % de $CO_2$. La composition de cette solution en mM est : NaCl 118 ; KCl 4,7 ; $CaCl_2$ 2,6 ; $KH_2PO_4$ 1,2 ; $MgCl_2$ 1,2 ; $NaHCO_3$ 25 ; glucose 11,7 ; acide ascorbique 1,0. L'oreillette droite est séparée du coeur. Sa base est attachée à un crochet monté sur un support et son apex à un autre petit crochet auquel un fil est accroché.
La préparation est transférée dans un bain à organe contenant 20 ml de solution de Krebs oxygénée à 32°C et le fil est connecté à un transducteur de force (Grass, modèle : FTO3C).
Une tension de repos de 0,5 g est appliquée à l'oreillette et les signaux de force contractile servent à déclencher un cardiotachomètre pour mesurer le rythme atrial. La force contractile et le rythme atrial sont enregistrés sur un polygraphe.

Après une période de stabilisation de 50 minutes, le tissu est soumis à une concentration de 0,01 µM d'isoprénaline puis est lavé à plusieurs reprises pendant 20 minutes.
Immédiatement après, la courbe concentration-effet complète est déterminée en additionnant au bain des concentrations de plus en plus fortes d'isoprénaline (demi-intervalles de l'échelle logarithmique) jusqu'à ce que le rythme atrial ne puisse plus augmenter davantage. Chaque concentration successive d'isoprénaline est ajoutée quand la réponse à la concentration précédente atteint un état stable apparent. A la fin de cette courbe concentration-effet, la préparation est lavée toutes les 10 minutes, pendant 30 minutes, pour lui laisser retrouver son rythme de battement minimal. Puis, une deuxième courbe de réponse aux concentrations est effectuée 30 minutes après avoir soumis la préparation aux différentes concentrations de *R*(+)-cicloprolol (0,3 ; 1 ; et 3 µM) ou de *S*(-)-cicloprolol (0,1 ; 0,3 et 1 µM).
Les modifications maximales du rythme atrial engendrées par chaque concentration d'isoprénaline sont exprimées en pourcentage par rapport à la réponse émise lors d'une concentration supramaximale d'isoprénaline. Ces valeurs sont utilisées pour construire la courbe concentration-effet dans un repère semi-logarithmique. Il faut noter que si le rythme initial au début de la seconde courbe concentration-effet diffère de celle de la 1ère courbe, la réponse maximale utilisée pour calculer les pourcentages est déterminée par la différence entre le rythme initial de l'oreillette avant l'initiation de la seconde courbe concentration-effet et le rythme atteint par l'oreillette après l'addition de la plus grande concentration d'isoprénaline lors de la première courbe de réponse.

Analyse des résultats

Le logarithme changé de signe de la concentration d'isoprénaline produisant 50 % de l'effet maximal ($pD_2$) est calculé pour chaque courbe de réponse avant et après chaque concentration de *S*(-) ou de *R*(+)-cicloprolol. Les valeurs logarithmiques (concentration ratio-1) sont portées sur l'ordonnée en fonction du logarithme négatif de la concentration d'antagoniste. (Arunlakshana et Schild, **Br. J. Pharmacol. 14, 48-54, 1959).**
Une droite des moindres carrés est tracée à partir de ces points expérimentaux et l'intersection avec l'abscisse est calculée avec ses intervalles de confiance. Cette valeur d'intersection est le $pA_2$ gui est défini comme le logarithme négatif de la concentration molaire d'antagoniste,en présence de laquelle il faut deux fois plus d'isoprenaline pour obtenir le même effet que celui observé en son absence.

Résultats

Les (*R*)(+)-cicloprolol et (*S*)(-)-cicloprolol déplacent vers la droite les courbes concentration-effet à l'isoprénaline de façon parallèle et sans changer la réponse maximale.
Le calcul de $pA_2$ (par la méthode de Schild) donne les valeurs suivantes :

7,44 (7,23 - 7,77) pour le (*S*)(-)-cicloprolol

6,62 (6,45 - 6,90) pour le (*R*)(+)-cicloprolol

Le (*S*)(-)-cicloprolol est 6,6 fois [antilog(7,44 - 6,62) = antilog(0,82) = 6,6] plus puissant que le (*R*)(+)-cicloprolol, comme antagoniste des récepteurs $\beta_1$ adrénergiques.

Ces résultats indiquent que le (*S*)(-)-cicloprolol est un antagoniste compétitif puissant des récepteurs $\beta_1$-adrénergiques présents dans l'oreillette isolée de cobaye.

**2. Propriétés β-agonistes partielles des isomères (*S*)(-) et (*R*)(+) du cicloprolol.**

L'activité β-agoniste partielle des deux énantiomères du cicloprolol, a été étudiée in vivo chez le Rat amyélé. Des rats (Spraque-Dawley ; Charles River, France) mâles de 250 g environ sont anesthésiés au pentobarbital sodique (55 mg/kg i.p.), amyélés puis placés sous respiration artificielle. La pression artérielle est captée par voie sanglante au niveau de la carotide et l'onde pulsatile alimente un tachographe gui permet de mesurer la fréquence cardiaque. Les composés sont injectés par voie intraveineuse au niveau de la veine fémorale. Ces rats amyélés présentent une fréquence cardiaque réduite d'environ 30 % par rapport à celle de rats intacts anesthésiés. L'activité agoniste partielle des énantiomères est déterminée par l'injection de doses cumulatives des énantiomères. On détermine la dose permettant une augmentation de la fréquence cardiaque de 50 battements par minute ($DE_{50}$) ainsi que le maximum de l'effet.

Résultats

L'augmentation maximale de fréquence cardiaque (effet agoniste partiel) obtenue respectivement avec le (*S*)(-)-cicloprolol et le (*R*)(+)-cicloprolol est de 107 ± 3 bat/min et de 113 ± 5 bat/min. Dans les mêmes conditions expérimentales l'isoprénaline, agoniste pur provoque des augmentations de la fréquence cardiaque d'environ 140 bat/min.
L'énantiomère (*S*)(-) présente une activité agoniste partielle:

$$DE_{50} = 4.6 \pm 0.1 \ \mu g/kg$$

Pour l'énantiomère (*R*)(+):

$$DE_{50} = 121.9 \pm 2.0 \ \mu g/kg.$$

Dans les mêmes conditions, pour l'isoprénaline

$$DE_{50} = 0,02 \ \mu g/kg.$$

Le (*S*)(-)cicloprolol possède donc une activité β-agoniste partielle.

Les isomères (*R*)(+) et (*S*)(-) du cicloprolol sont donc utiles dans le domaine cardiovasculaire. Plus spécifiquement l'isomère (*S*)(-) du cicloprolol peut être utilisé pour le traitement de l'insuffisance cardiaque.

**3. Activité antiglaucome des isomères (*S*)(-) et (*R*)(+) du cicloprolol.**

Les composés de l'invention ont été testés pour leur activité antiglaucome.
La pression intraoculaire (PIO) aigue d'un jour a été évaluée sur des yeux normotendus et sur des yeux hypertendus chez le singe; elle est déterminée à l'aide d'un pneumatonographe après une légère anesthésie de la cornée par de la proparacaïne.
Les yeux droits de tous les singes ont été traités par trabeculoplastie au laser pendant plusieurs mois avant l'expérience, afin de développer une hypertension oculaire. Les animaux sont entrainés à s'asseoir dans des chaises de contention et sont conditionnés pour accepter les mesures de la PIO.
Après chaque mesure de la PIO, l'anesthésiant résiduel est éliminé par lavage avec une solution saline.
Après mesure de la PIO de référence, le composé à tester ou le solvant est administré, en deux apports de 25 μl, aux deux yeux de 6 singes Cynomolgus. Les mesures de la PIO sont réalisées 1, 3, et 7 heures après l'administration.

Les résultats montrent que les composés de l'invention peuvent être utilisés pour le traitement du glaucome.

Les composés de l'invention peuvent être proposés sous toute forme appropriée, en association avec tout excipient adéquat, par exemple, sous la forme de comprimés, de gélules, de solutions ou de suspensions.

**Revendications**

1. Isomères (*R*)(+) et (*S*)(-) du 1-[4-[2-(cyclopropylméthoxy)éthoxy]phénoxy]-3-[(1-méthyléthyl)amino]propan-2-ol et leurs sels pharmaceutiquement acceptables.

2. (*S*)-(-)-1-[4-[2(cyclopropylméthoxy)éthoxy]phénoxy]-3-[(1-méthyléthyl)amino]propan-2-ol et ses sels d'addition aux acides pharmaceutiquement acceptables.

3. Médicament caractérisé en ce qu'il contient le (*R*)(+) ou le (*S*)-(-)-1-[4-[2-(cyclopropylméthoxy)éthoxy]phénoxy]-3-[(1-méthyléthyl)amino]-propan-2-ol ou un de leurs sels d'addition aux acides pharmaceutiquement acceptables.

**4.** Médicament caractérisé en ce qu'il contient le (*S*)-(-)-1-[4-[2-(cyclopropylméthoxy)éthoxy]phénoxy]-3-[1-méthyléthyl)amino]propan-2-ol ou un de ses sels d'addition aux acides pharmaceutiquement acceptables.

**5.** Composition pharmaceutique caractérisée en ce qu'elle contient le (*R*)(+) ou le (*S*)-(-)-1-[4-[2(cyclopropylméthoxy)éthoxy]phénoxy]-3-[(1-méthyléthyl)amino]propan-2-ol ou un de leurs sels d'addition aux acides pharmaceutiquement acceptables, en association avec tout excipient approprié.

**6.** Composition pharmaceutique caractérisée en ce qu'elle contient le (*S*)-(-)-1-[4-[2(cyclopropylméthoxy)éthoxy]phénoxy]-3-[(1-méthyléthyl)amino]propan-2-ol ou un de ses sels d'addition aux acides pharmaceutiquement acceptables, en association avec tout excipient approprié.

**7.** Procédé de préparation du (*S*)-(-)-1-[4-[2(cyclopropylméthoxy)éthoxy]phénoxy]-3-[(1-méthyléthyl)amino]propan-2-ol, procédé caractérisé en ce qu'on fait réagir le (5*S*)-3-(1-méthyléthyl)-2-phényloxazolidine-5-méthanol sous forme de méthylbenzènesulfonate avec le 4-[3-(cyclopropylméthoxy)éthoxy]phénol.

**8.** Procédé de préparation du (*R*)-(+)-1-[4-[2(cyclopropylméthoxy)éthoxy]phénoxy]-3-[(1-méthyléthyl)amino]propan-2-ol, procédé caractérisé en ce qu'on fait réagir le 4-méthylbenzènesulfonate de [(*R*)-2,2-diméthyldioxolan-4-yl]méthyle avec le 4-[2-(cyclopropylméthoxy)éthoxy]phénol puis on transforme le composé intermédiaire en (*R*)-3-[4-[2-(cyclopropylméthoxy)éthoxy]phénoxy]propane-1,2-diol que l'on transforme en (*R*)-2-[[4-[2-(cyclopropylméthoxy)éthoxy]phénoxy]méthyl]oxirane, composé que l'on fait réagir avec l'isopropylamine.

**9.** Procédé de préparation enzymatique du (*S*)-(-)-1-[4-[2(cyclopropylméthoxy)éthoxy]phénoxy]-3-[(1-méthyléthyl)amino]propan-2-ol et du (*R*)-(+)-1-[4-[2(cyclopropylméthoxy)éthoxy]phénoxy]-3-[1-(méthyléthyl)amino]propan-2-ol, caractérisé en ce que l'on condense le 4-[2-(cyclopropylméthoxy)éthoxy]phénol avec l'épichlorhydrine en milieu basique pour obtenir un mélange que l'on traite par de l'acide chlorhydrique pour obtenir du 1-chloro-3-[4-[2-(cyclopropylméthoxy)éthoxy]phénoxy]propan-2-ol, composé que l'on acétyle

. soit dans des conditions classiques pour obtenir l'acétate de 1-chloro-3-[4-[2-(cyclopropylméthoxy)éthoxy]phénoxy]prop-2-yle que l'on hydrolyse en présence de lipase pour obtenir un mélange de (*S*)-1-chloro-3-[4-[2-(cyclopropylméthoxy)éthoxy]phénoxy]propan-2-ol 2(*S*) et d'acétate de (*R*)-1-chloro-3-[4-[2-(cyclopropylméthoxy)éthoxy]phénoxy]prop-2-yle 3(*R*), que l'on peut séparer par chromatographie sur colonne de gel de silice, ou par traitement par de l'anhydride succinique en présence de triéthylamine,

. soit par une lipase en présence d'un agent acylant pour obtenir un mélange de (*R*)-1-chloro-3-[4-[2-(cyclopropylméthoxy)éthoxy]phénoxy]propan-2-ol 2(R) et d'acétate de (*S*)-1-chloro-3-[4-[2-(cyclopropylméthoxy)éthoxy]phénoxy]prop-2-yle 3(S) qui sont séparés par chromatographie sur colonne de gel de silice,

puis on transforme respectivement les composés 2(*R*) et 2(*S*) (*S*)-cicloprolol et en (*R*)-cicloprolol par formation d'un époxyde en milieu basique que l'on fait réagir avec de l'isopropylamine et on hydrolyse les composés 3(*R*) et 3(*S*) avant de les transformer respectivement en (*S*)-cicloprolol et en (*R*)-cicloprolol par formation d'un époxyde en milieu basique que l'on fait réagir avec de l'isopropylamine.

**10.** Procédé selon la revendication 9, caractérisé par le fait que la lipase est issue de Pseudomonas fluorescens.

**Revendications pour les Etats contractants suivants: ES, GR**

**1.** Procédé de préparation du (*S*)-(-)-1-[4-[2(cyclopropylméthoxy)éthoxy]phénoxy]-3-[(1-méthyléthyl)amino]propan-2-ol, procédé caractérisé en ce qu'on fait réagir le (5*S*)-3-(1-méthyléthyl)-2-phényloxazolidine-5-méthanol sous forme de méthylbenzènesulfonate avec le 4-[3-(cyclopropylméthoxy)éthoxy]phénol.

**2.** Procédé de préparation du (*R*)-(+)-1-[4-[2(cyclopropylméthoxy)éthoxy]phénoxy]-3-[(1-méthyléthyl)amino]propan-2-ol, procédé caractérisé en ce qu'on fait réagir le 4-méthylbenzènesulfonate de [(*R*)-2,2-diméthyldioxolan-4-yl]méthyle avec le 4-[2-(cyclopropylméthoxy)éthoxy]phénol puis on transforme le composé intermédiaire en (*R*)-3-[4-[2-(cyclopropylméthoxy)éthoxy]phénoxy]propane-1,2-diol que l'on transforme en (*R*)-2-[[4-[2-(cyclopropylméthoxy)éthoxy]phénoxy]méthyl]oxirane, composé que l'on fait réagir avec l'isopropylamine.

3. Procédé de préparation enzymatique du (*S*)-(-)-1-[4-[2(cyclopropylméthoxy)éthoxy]phénoxy]-3-[(1-méthyléthyl)amino]propan-2-ol et du (*R*)-(+)-1-[4-[2(cyclopropylméthoxy)éthoxy]phénoxy]-3-[(1-méthyléthyl)amino]propan-2-ol, caractérisé en ce que l'on condense le 4-[2-(cyclopropylméthoxy)éthoxy]phénol avec l'épichlorhydrine en milieu basique pour obtenir un mélange que l'on traite par de l'acide chlorhydrique pour obtenir du 1-chloro-3-[4-[2-(cyclopropylméthoxy)éthoxy]phénoxy]propan-2-ol, composé que l'on acétyle
   . soit dans des conditions classiques pour obtenir l'acétate de 1-chloro-3-[4-[2-(cyclopropylméthoxy)éthoxy]phénoxy]prop-2-yle que l'on hydrolyse en présence de lipase pour obtenir un mélange de (*S*)-1-chloro-3-[4-[2-(cyclopropylméthoxy)éthoxy]phénoxy]propan-2-ol 2(*S*) et d'acétate de (*R*)-1-chloro-3-[4-[2-(cyclopropylméthoxy)éthoxy]phénoxy]prop-2-yle 3(*R*), que l'on peut séparer par chromatographie sur colonne de gel de silice ou par traitement par de l'anhydride succinique en présence de triéthylamine,
   . soit par une lipase en pésence d'un agent acylant pour obtenir un mélange de (*R*)-1-chloro-3-[4-[2-(cyclopropylméthoxy)éthoxy]phénoxy]propan-2-ol 2(*R*) et d'acétate de (*S*)-1-chloro-3-[4-[2-(cyclopropylméthoxy)éthoxy]phénoxy]prop-2-yle 3(*S*) qui sont séparés par chromatographie sur colonne de gel de silice,

   puis on transforme respectivement les composés 2(*R*) et 2(*S*) en (*S*)-cicloprolol et en (*R*)-cicloprolol par formation d'un époxyde en milieu basique que l'on fait réagir avec de l'isopropylamine et on hydrolyse les composés 3(*R*) et 3(*S*) avant de les transformer respectivement en (*S*)-cicloprolol et en (*R*)-cicloprolol par formation d'un époxyde en milieu basique que l'on fait réagir avec de l'isopropylamine.

4. Procédé selon la revendication 3, caractérisé par le fait que la lipase est issue de Pseudomonas fluorescens.

Office européen des brevets

# RAPPORT DE RECHERCHE EUROPEENNE

Numero de la demande

EP 92 40 0267

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A,D | FR-A-2 330 383 (SYNTHELABO)<br>* revendications; exemple 1 * | 1-10 | C07C217/34<br>C12N9/18 |
| X | JOURNAL OF CHROMATOGRAPHY<br>vol. 452, 1988, AMSTERDAM<br>pages 477 - 483;<br>A.M. KRSTULOVIC ET AL.: 'DIRECT ENATIOMERIC SEPARATION OF BETAXOLOL WITH APPLICATIONS TO ANALYSIS OF BULK DRUG AND BIOLOGICAL SAMPLES'<br>* le document en entier * | 1-6 | |
| X | JOURNAL OF CHROMATOGRAPHY<br>vol. 539, 1991, AMSTERDAM<br>pages 55 - 69;<br>C.R. LEE ET AL.: 'LIQUID AND HIGH-PRESSURE CARBON DIOXIDE CHROMATOGRAPHY OF BETA- BLOCKERS'<br>*Résumé*<br>* page 62 *<br>& 11TH INTERNATIONAL SYMPOSIUM ON BIOMEDICAL APPLAICATIONS OF CHROMATOGRAPHY AND ELECTROPHORESIS<br>24 Avril 1990, TALLINN | 1-6 | DOMAINES TECHNIQUES RECHERCHES (Int. Cl.5 )<br>C07C |

Le présent rapport a été établi pour toutes les revendications

| Lieu de la recherche | Date d'achèvement de la recherche | Examinateur |
|---|---|---|
| LA HAYE | 07 MAI 1992 | SANCHEZ Y GARCIA J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)